# EUROPÄISCHE PATENTANMELDUNG

(11) **EP 3 708 144 A1**
(43) Veröffentlichungstag der Anmeldung: **16.09.2020**
(21) Anmeldenummer: 19000127.1
(22) Anmeldetag: 11.03.2019
(51) Int. Cl.: A61K 8/25, A61Q 5/00, A61K 8/60

(54) **HAARPFLEGEMETHODE ZUR REGENERATION VON HAARFOLLIKELN**

(71) Anmelder: Podolski, Kevin, 10247 Berlin (DE)
(72) Erfinder: Podolski, Kevin, 10247 Berlin (DE)

(57) **Zusammenfassung**

Spezifisches Anwendungsverfahren eines nicht-oberflächenaktiven Zuckers zur Haarpflege, Regeneration und Verhinderung einer Miniaturisierung von Haarfollikeln

## Beschreibung

### Technisches Gebiet

Die vorliegende Erfindung betrifft das Anwendungsverfahren einer Substanz, das in dieser Kombination zu einer Regeneration von Haarfollikeln beiträgt

### Stand der Technik

Haarpflegemittel sind in unterschiedlichen Ausführungen und für unterschiedliche Anwendungen seit vielen Jahren auf dem Markt und dienen hauptsächlich entweder der Reinigung oder der Pflege der vorhandenen Haare.

Ein bekanntes Haarpflegeverfahren stellt dabei die unter den Veröffentlichungsnummern:
EP 2282816 B1, US 8,329,151 B2, A61 Q 5/00, A61 K 8/60, G01 N 33/68 erteilte Anwendung der auch hier zugrunde liegenden Substanz dar.

Das darin einheitlich beschriebene Verfahren besteht bevorzugt aus den Schritten:
i) Aufbringen einer Zusammensetzung eines nichtoberflächenaktiven, veresterten Zuckers
ii) gefolgt von dem Waschen der Haare, innerhalb von einer Stunde, stärker bevorzugt, innerhalb von 5 Minuten, in herkömmlicher Weise, unter Verwendung eines handelsüblichen Shampoos um durch diese Anwendung ein Entwirren der Haare zu verbessern.

### Aufgabe

Wirksame Substanzen zur Regeneration von miniaturisierten und zur zur Generation von Haarfollikeln sind nicht bekannt oder auf dem Markt erhältlich.

Die Erfindung erzielt erst durch Anwendung eines spezifischen Verfahrens, der unter den Veröffentlichungs-. nummern EP 2282816 B1, US 8,329,151 B2, A61 Q 5/00, A61 K 8/60, G01 N 33/68 bekannten Substanz einen positiven Einfluss auf den Pflegezustand der Haare und die Haarfollikel

### Darstellung der Erfindung

Es ist bevorzugt, denn das Verfahren zur Haarbehandlung folgende Schritte umfasst:
i) Aufbringen einer Verbindung, die ein Silicon mit einem tensidfreien, veresterten Zucker, der bei 20 Grad Celsius nicht kristallin ist, der Saccharoseacetatisobutyrat ist, vorzugsweise unter Verwendung der unter den Veröffentlichungsnummern EP 2282816 B1, US 8,329,151 B2, A61 Q 5/00, A61 K 8/60, G01 N 33/68 bekantten Substanz
ii) Ausspühlen oder Abwaschen der Substanz, nachdem diese insgesamt mehr als eine Stunde, auch bei Stückelung der Gesamteinwirkzeit, aufgetragen war und regelmäßig, täglich, über einen Zeitraum von mindestens 3 Monaten, wiederholt wird

Eine zusätzliche Anwendung handelsüblicher Shampoos ist nicht zwingend erforderlich.

## Patentansprüche

1. Verfahren zur Pflege von Haaren und zur Regeneration von Haarfollikeln durch Aufbringen einer Substanz

2. Verfahren nach Anspruch 1., das die folgenden Schritte aufweist:
i) Aufbringen einer Substanz, die Saccharoseacetatisobutyrat und Silicon aufweist, auf das Haar
ii) Verbleiben der Substanz auf dem Haar, über einen Gesamtzeitraum von mehr als einer Stunde, auch bei Stückelung des Gesamtzeitraums
iii) Ausspühlen oder Auswaschen der Substanz
